(19) Europäisches Patentamt
European Patent Office
Office européen des brevets

(11) Publication number: **0 089 105**
**B1**

(12) # EUROPEAN PATENT SPECIFICATION

(45) Date of publication of patent specification: **18.09.85**

(51) Int. Cl.⁴: **C 10 K 3/04**

(21) Application number: **83300388.2**

(22) Date of filing: **26.01.83**

(54) **Multi stage methanation.**

(30) Priority: **12.03.82 GB 8207245**

(43) Date of publication of application:
**21.09.83 Bulletin 83/38**

(45) Publication of the grant of the patent:
**18.09.85 Bulletin 85/38**

(84) Designated Contracting States:
**BE DE FR IT NL**

(56) References cited:
**DE-A-2 722 502**
**GB-A-2 060 686**

(73) Proprietor: **British Gas Corporation**
**Rivermill House 152 Grosvenor Road**
**London SW1V 3JL (GB)**

(72) Inventor: **Tart, Keith Raymond**
**92 Buffery Road**
**Dudley West Midlands (GB)**

(74) Representative: **Wallace, Walter**
**British Gas Corporation Patents Department 326**
**High Holborn**
**London, WC1V 7PT (GB)**

**EP 0 089 105 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the production of methane containing gases and, more particularly to the production of fuel gases suitable for use as substitute natural gas (SNG) from synthesis gases obtained by the gasification of carbonaceous fuels such as oils and coal.

Considerable research has and is being carried out in the field of coal gasification, an example of which is the gasification of coal under ash slagging conditions. The primary constituents of the synthesis gas from a fixed bed slagging gasifier are carbon monoxide and hydrogen. In order to increase the methane content of the gas to produce SNG the hydrogen and carbon monoxide may be catalytically methanated according to the reaction—

$$3H_2+CO \rightarrow CH_4+H_2O$$

In order to provide hydrogen and carbon monoxide concentrations to give molar ratios of the order of 3:1 ($H_2$:CO), the composition of the primary synthesis will normally have to be adjusted.

The necessary adjustment can be achieved by subjecting the synthesis gas to the CO shift conversion wherein carbon monoxide is reacted with large amounts of steam in the presence of a catalyst, to produce hydrogen and carbon dioxide.

The earlier coal gasification processes often produced a synthesis gas in which there was a large excess of unreacted steam and thus it was convenient to effect the CO shift conversion. However, many of the more modern processes yield a synthesis product gas which contains large amounts of carbon monoxide (typically in excess of 30% by volume on a dry basis) and small amounts of indigenous steam. Thus in order to adjust the hydrogen to carbon monoxide ratio prior to methanation large amounts of reactant steam have to be imported into the process. The energy required to raise this imported steam is expensive and results in an economic burden on the process and a consequent reduction in overall thermal efficiency.

In our UK Patent Specification No. 1544245 there is described a process for upgrading high CO-containing primary synthesis gases without recourse to an intermediate shift conversion wherein *inter alia*, the synthesis gas is simultaneously heated and saturated with water, the hot saturated gas being thereafter divided into two streams. A reactant stream comprising the first of the two streams is subjected to a catalytic CO shift conversion-methanation reactions to produce a methanation product which is blended with the second of the two saturated streams and thereafter the blended stream is subjected to catalytic methanation to produce a second methanation product. A portion of the second methanation product is recycled and blended with the first saturated gas to form the reactant stream.

We now propose a process for methanating high CO containing synthesis gases which eliminates the need for gas recycle, as required in our aforesaid UK Patent No. 1544245.

According to the present invention there is provided a process for producing methane rich gases by the catalytic CO shift conversion-methanation of a synthesis gas containing hydrogen and at least 30% by volume (dry basis) of carbon monoxide which process includes the steps of:

(i) dividing the synthesis gas into a first minor stream and a second major stream,

(ii) mixing said first minor stream with steam to form a preliminary reactant mixture, the temperature of which is within the range of 200 to 450°C and subjecting said preliminary, reactant mixture to at least one catalytic CO shift conversion-methanation to produce a preliminary reaction product,

(iii) subjecting at least a portion of said second major stream to simultaneous heating and saturation with water to form a first saturated product,

(iv) mixing said preliminary reaction product with at least a portion of said first saturated product to produce a first stage reactant stream, the temperature of which is within the range of 200 to 450°C,

(v) subjecting said first stage reactant to a first stage catalytic CO shift conversion-methanation to form a first stage reaction product,

(vi) subjecting any remaining portion of said second stream to simultaneous heating and saturation with water to form a second saturated product,

(vii) mixing said first stage reaction product with at least a portion of said second saturated product to form a second stage reactant stream, the temperature of which is within the range of 200 to 450°C.

(viii) subjecting said second stage reactant stream to a second stage catalytic methanation to form a second stage reaction product.

Although satisfactory CO shift conversion-methanation may sometimes be achieved by utilising a single methanation reactor in each of the methanation stages, beneficial results may be obtained by utilising a plurality of reactors, eg at least three, arranged in series in each stage and by dividing the saturated product streams between each stage. The advantage of such an arrangement is that the proportion of synthesis gas required for the first (minor) stream in order to prevent excessively high outlet temperatures from the first and second methanation stages, which may cause catalyst sintering, is reduced. This results in the flow-rate of the second (the major) stream being increased with beneficial effect on process efficiency owing to the increased use of saturators for process steam addition.

Furthermore it is preferred to subject the second stage reaction product which is at a temperature of not greater than 500°C to at least one further CO shift conversion-methanation reaction without the further addition of second saturated product in order to reduce the CO and $H_2$ content of the previous stage product further still. In order to maximise the methane yield it may also be preferred to remove carbon dioxide and, optionally water vapour from the second stage methanation product prior to the further methanation.

It may be desirable to carry out some CO shift conversion prior to the preliminary methanation of the minor synthesis gas stream in order to increase the $H_2$:CO ratio to alleviate problems of carbon deposition. It should be noted that this preliminary CO shift conversion step is not essential to the operation of the process of the invention.

In the operation of the process of the invention it is preferred to divide the synthesis gas stream which contains at least 30% typically at least 55% by volume (dry basis) of carbon monoxide such that the first minor stream comprises from 5 to 30% by volume, more preferably about 20% by volume, of the synthesis gas.

The temperature of the water used to saturate and heat the second stream portions preferably ranges from 100°C to 300°C. Obviously in order to maintain the water in the liquid phase prior to contact with the second stream portions, the pressure of the system has to be sufficiently high and in any event the temperature must be below the critical temperature. The water used for heating and saturating may itself be heated by direct or indirect heat exchange with hot gases throughout the entire process train. However, it is preferred that at least some of the heat requirement be obtained by heat exchange with the product gas from the final methanation stage or stages of the process stream.

The temperature of the reactant mixtures for each methanation stage should be within the range of 200 to 450°C. Since the outlet temperature from the previous methanation stage may be in excess of 500° some cooling of the previous methanation product is necessary. This may be achieved in part by direct heat exchange on admixture with the portion of saturated product or, by cooling the methanation products prior to admixture with the saturated product. This cooling may be achieved by indirect heat exchange with, for example, water to be used for heating and saturating the second stream or steam raising boilers.

The amount of water required to saturate the second stream will vary depending upon, amongst other things, the indigenous steam content of the feed gas and the steam to dry gas ratio required for effective methanation. However, it is preferred that the steam to gas ratio (on a dry gas basis) of the heated, saturated product should be from 0.1—1.0:1.

The process of the invention may be carried out at substantially the same pressures as that employed for the primary gasification reactions and thus can be carried out at pressures ranging from 3 bar to 140 bar.

The use of gas compressors within the process stream of train is usually necessary and we have found that in view of the split stream configuration of the process train it is not necessary to compress all of the synthesis gas feed streams.

Effective pressurisation can be achieved by compressing the minor synthesis gas stream prior to either the CO shift conversion stage, when used, or to the preliminary methanation stage. Additionally it may be desirable to compress the first portion of the second stream prior to heating and saturation. The combined power requirement of these small compressors is considerably less than a gas recycle compressor commonly used in other processes.

The process of the present invention is particularly efficient for the methanation of synthesis gases which contain low amounts of indigenous steam and have carbon monoxide contents in excess of 30% by volume (dry basis) and the process may be used with even greater advantage for synthesis gases containing in excess of 55% by volume (dry basis) of carbon monoxide.

The process of the invention will be illustrated in greater detail with reference to the following Example and the accompanying drawing which is a flow-sheet showing one embodiment of the invention.

A synthesis gas having the composition shown in Table 1, column A, enters the process train via line 1. The gas stream is divided into a major stream (2) and a minor stream (3), the minor stream comprising 22% of the total feed. The temperature and pressures of the inlet feed stream are 30°C and 28 bar respectively. The pressure of stream 3 is increased to 39 bar by compressor 4 and steam at a temperature of 405°C from line 5 is admixed with the compressed gas to produce a reactant mixture which is passed into a catalytic CO shift conversion reactor 6. The ratio of steam to feed gas in the shift reactor is about 2:1. During the shift conversion the carbon monoxide content is reduced to about 4% and the carbon dioxide and hydrogen contents are increased to about 37% and 50% respectively.

The reaction product from 6 is then passed into the preliminary methanator 7, after temperature adjustment (not shown).

The major synthesis gas stream 2 is subdivided into two portions 21 and 22. Stream 21 is subjected to compression in compressor 8 from 28 bar to 35 bar and thereafter fed into the base of saturator 9 to produce a heated saturated product whose temperature is 138°C and has a steam to dry gas ratio of 0.1:1.0.

Saturated hot gas is removed from the top of the saturator via line 17.

Product from the methanator unit 7 is cooled (not shown) and admixed with a portion of the saturated product from line 17 to form a first

stage methanation reactant mixture, the temperature of which is 300°C.

The first stage methanator consists of three reactors, 10, 11 and 12 arranged in series. A portion of the saturated product in line 17 is admixed with each methanation reaction product from 10 and 11 prior to reaction in the succeeding reactors. The temperature of the reactant at the inlet of each reactor is 300°C. The first stage methanation product from the final reactor 12 in the series has composition shown in column B of Table 1.

Synthesis gas in line 22 is fed to saturator 19 and the heated saturated gas is removed via line 18.

The first stage methanation reaction product from reactor 12 is admixed with a portion of the second saturated product from line 18 to form a second stage methanation reactant mixture which is fed to the inlet of a three reactor (13, 14 and 15), series arranged, second stage methanator. The temperature at the inlet to each of reactors 13 and 14 is 300°C and to each of the products from those reactors is added a further portion of the second saturated product from line 18. The composition of the gas existing from the final reactor (15) of the second stage methanation is shown in column C of Table 1.

The reaction product from 15 is then fed, without further additions, to the further methanation stage 16. The gas exiting from the reactor 16 has a composition shown in column D of Table 1 and after carbon dioxide removal according to conventional techniques has a composition suitable for use as substitute natural gas.

The water used in the heater-saturators 9 and 19 is heated by direct heat exchange (not shown) with the product gas from reactor 16, wherein the product gas is contacted directly with liquid water in a dehumidifier.

TABLE 1

| Gas stream | A | B | C | D |
|---|---|---|---|---|
| Dry gas composition (mol%) | | | | |
| CO | 53.23 | 2.03 | 0.16 | 0.03 |
| $CO_2$ | 7.96 | 51.53 | 55.50 | 55.98 |
| $H_2$ | 27.30 | 10.27 | 2.10 | 0.85 |
| $CH_4$ | 9.97 | 35.00 | 40.97 | 41.85 |
| $C_2H_6$ | 0.45 | — | — | — |
| $N_2$ | 0.95 | 1.17 | 1.27 | 1.29 |

In comparing the process of the present invention with a process of similiar size, with, for example, the process described in UK 1544245 we have found that both the catalyst requirement and total catalytic reactor vessel volume is about 15% less for the process of the present invention.

For process trains capable of producing $3.1\times10^6$ nm³/day of SNG the gas compression duties are very moderate ($6.5\times10^6$ nm³/day, 2700 KW) for processes of the present invention compared with the duty of the recycle compressor ($26.5\times10^6$ nm³/day, 15700 KW) required for a comparable train according to UK 1544245. This is equivalent to an energy saving of about 5% of the product gas thermal output, representing an increase of about 3% on overall thermal efficiency for SNG production.

## Claims

1. A process for producing methane-rich gases by the catalytic CO shift conversion methanation of a synthesis gas containing hydrogen and at least 30% by volume (dry basis) of carbon monoxide, which process includes the steps of:

(i) dividing the synthesis gas into a first minor stream and a second major stream,

(ii) mixing said first stream with steam to form a preliminary reactant, the temperature of which is within the range of 200 to 450°C and subjecting said preliminary reactant mixture to catalytic CO shift conversion-methanation to produce a preliminary reaction product,

(iii) subjecting at least a portion of said second major stream to simultaneous heating and saturation with water to form a first saturated product,

(iv) mixing said preliminary reaction product with at least a portion of said first saturated product to produce a first stage reactant stream, the temperature of which is within the range of 200 to 450°C.

(v) subjecting said first stage reactant to a first stage catalytic CO shift conversion-methanation to form a first stage methanation reaction product,

(vi) subjecting any remaining portion of said second stream to simultaneous heating and saturation with water to form a second saturated product,

(vii) mixing said first stage reaction product with at least a portion of said second saturated product to form a second stage reactant stream, the temperature of which is within the range of 200 to 450°C,

(viii) subjecting said second stage reactant stream to second stage catalytic methanation to form a second stage methanation reaction product,

2. A process as claimed in Claim 1 wherein said first stage methanation comprises a plurality of methanation stages, in series, the product from the preceding stage being admixed with a portion of said first saturated product to form a reactant mixture for the subsequent stage, and wherein the temperature of each of said reactant mixtures is within the range of 200 to 450°C.

3. A process as claimed in Claim 1 wherein said

second stage methanation comprises a plurality of methanation stages, in series, the product from the preceding stage being admixed with a portion of said second saturated product to form a reactant mixture for the subsequent stage, and wherein the temperature of each of said reactant mixtures is within the range of 200 to 450°C.

4. A process as claimed in Claim 1 wherein said second stage reaction product is subjected to at least one further catalytic methanation reaction, the temperature of said second stage reaction product, immediately prior to said further methanation being not greater than 500°C, to form a final reaction product.

5. A process as claimed in Claim 4 wherein carbon dioxide and, optionally, water vapour are removed from the second stage product prior to said further catalytic methanation.

6. A process as claimed in any one of the preceding claims wherein said first stream is subjected to CO shift conversion prior to preliminary methanation.

7. A process as claimed in any one of the preceding claims wherein said first stream comprises from 5 to 30% by volume of said synthesis gas.

8. A process as claimed in Claim 7 wherein said first stream comprises about 20% by volume of synthesis gas.

9. A process as claimed in any one of the preceding claims wherein the temperature of the water used to simultaneously heat and saturate said second stream portions is from 100° to 300°C.

10. A process as claimed in Claim 9 wherein at least a portion of the water is heated by direct or indirect heat exchange with streams within the process train.

11. A process as claimed in Claim 10 wherein said water is heated by direct or indirect heat exchange with methanation reaction product.

12. A process as claimed in any one of the preceding claims wherein the first and second stage methanation reaction products are cooled prior to admixture with saturated product.

13. A process as claimed in any one of the preceding claims wherein the steam/dry gas ratio of the saturated products are from 01.1—1.0:1.

14. A process as claimed in any one of the preceding claims wherein the carbon monoxide content of said synthesis gas is at least 55% by volume (dry basis).

15. A process as claimed in any one of the preceding claims wherein said methanation reactions are carried out at a pressure of from 3 bar to 140 bar.

16. A process as claimed in any one of the preceding claims wherein said first stream is compressed prior to effecting the CO shift conversion or preliminary methanation.

17. A process as claimed in any one of the preceding claims wherein said portion of said second stream forming said first saturated product is compressed prior to said simultaneous heating and saturation.

18. A process as claimed in any one of the preceding claims wherein at least three methanation reactors are provided in each stage.

**Patentansprüche**

1. Verfahren zur Erzeugung methanreicher Gase durch die katalytische CO-Verschiebungs-Konversionsmethanierung eines Synthesegases, das Wasserstoff und mindestens 30 Volumenprozent (Trockenbasis) Kohlenmonoxid enthält, gekennzeichnet durch folgende Verfahrensschritte:

(I) Aufteilung des Synthesegases in einen ersten Nebenstrom und einen zweiten Hauptstrom,

(II) Mischen des ersten Stromes mit Dampf, um ein vorläufiges Reaktionsmittel zu bilden, dessen Temperatur im Bereich von 200—450°C liegt, und Unterwerfung dieses vorläufigen Reaktionsmittelgemisches der katalytischen CO-Verschiebungs-Konversionsmethanierung, um ein vorläufiges Reaktionsprodukt zu erzeugen,

(III) Unterwerfung von mindestens einem Teil des zweiten Hauptstroms einem gleichzeitigen Erhitzen und Sättigen mit Wasser, um ein erstes gesättigtes Produkt zu bilden,

(IV) Mischen des vorläufigen Reaktionsproduktes mit mindestens einem Teil des ersten gesättigten Produktes, um einen Erststufen-Reaktionsmittelstrom zu erzeugen, dessen Temperatur im Bereich von 200—450°C liegt,

(V) Unterwerfung dieses Erststufen-Reaktionsmittels einer katalytischen Erststufen-CO-Verschiebungs-Konversionsmethanierung, um ein Erststufen-Methanierungs-Reaktionsprodukt zu bilden,

(VI) Unterwerfung des übrigen Teils des Zweitstroms einer gleichzeitigen Erhitzung und Sättigung mit Wasser, um ein zweites gesättigtes Produkt zu bilden,

(VII) Mischen des Erststufen-Reaktionsproduktes mit mindestens einem Teil des zweiten gesättigten Produktes, um einen Zweitstufen-Reaktionsmittelstrom zu bilden, dessen Temperatur im Bereich von 200—450°C liegt,

(VIII) Unterwerfung des Zweitstufen-Reaktionsmittelstromes einer katalytischen Zweitstufen-Methanierung, um ein Zeitstufen-Methnierungs-Reaktionsprodukt zu bilden.

2. Verfahren nach Anspruch 1, dadurch gekennzeichnet, daß die Erststufen-Methnierung eine Vielzahl von Methanierungsstufen in Reihenschaltung aufweist, wobei das Produkt der vorhergehenden Stufe einem Teil des ersten gesättigten Produktes beigemischt wird, um ein Reaktionsmittelgemisch für die nachfolgende Stufe zu bilden, und daß die Temperatur von jedem dieser Reaktionsmittelgemische im Bereich von 200—450°C liegt.

3. Verfahren nach Anspruch 1, dadurch gekennzeichnet, daß die Zweitstufen-Methanierung eine Vielzahl von Methanierungsstufen in Reihenschaltung aufweist, wobei das Produkt der vorhergehenden Stufe einem Teil des zweiten gesättigten Produktes beigemischt wird, um ein Reaktionsmittelgemisch für die nachfolgende Stufe zu bilden, und daß die Tpmperatur von jedem dieser Reaktionsmittelgemische im Bereich von 200—450°C liegt.

4. Verfahren nach Anspruch 1, dadurch gekennzeichnet, daß das Zweitstufen-Reaktionsprodukt mindestens einer weiteren katalytischen Methanierungsreaktion unterworfen wird, wobei die Temperatur dieses Zweitstufen-Reaktionsproduktes unmittelbar vor dieser weiteren Methanierung nicht höher als 500°C liegt, um ein endgültiges Reaktionsprodukt zu bilden.

5. Verfahren nach Anspruch 4, dadurch gekennzeichnet, daß Kohlendioxid und wahlweise Wasserdampf dem Zweitstufenprodukt vor der weiteren katalytischen Methanierung entzogen werden.

6. Verfahren nach einem der Ansprüche 1 bis 5, dadurch gekennzeichnet, daß der Erststrom der CO-Verschiebungs-Konversion vor der vorläufigen Methanierung ausgesetzt wird.

7. Verfahren nach einem der Ansprüche 1 bis 6, dadurch gekennzeichnet, daß der Erststrom 5 bis 30 Volumenprozent des Synthesegases aufweist.

8. Verfahren nach Anspruch 7, dadurch gekennzeichnet, daß der Erststrom etwa 20 Volumenprozent des Synthesegases aufweist.

9. Verfahren nach einem der Ansprüche 1 bis 8, dadurch gekennzeichnet, daß die Temperatur des Wassers, welches zum gleichzeitigen Erhitzen und Sättigen der Zweitstrom-Anteile verwendet wird, zwischen 100 und 300°C liegt.

10. Verfahren nach Anspruch 9, dadurch gekennzeichnet, daß mindestens ein Teil des Wassers durch direkten oder indirekten Wärmeaustausch mit Strömen innerhalb des Verfahrenszugs erhitzt wird.

11. Verfahren nach Anspruch 10, dadurch gekennzeichnet, daß Wasser durch direkten oder indirekten Wärmeaustausch mit dem Methanierungs-Reaktionsprodukt erhitzt wird.

12. Verfahren nach einem der Ansprüche 1 bis 11, dadurch gekennzeichnet, daß die Erst- und Zweitstufen-Methanierungs-Reaktionsprodukte vor der Beimischung zum gesättigten Produkt gekühlt werden.

13. Verfahren nach einem der Ansprüche 1 bis 12, dadurch gekennzeichnet, daß das Dampf/Trockengas-Verhältnis der gesättigten Produkte im Bereich 0,1:1—1,0:1 liegt.

14. Verfahren nach einem der Ansprüche 1 bis 13, dadurch gekennzeichnet, daß der Kohlenmonoxidgehalt des Synthesegases mindestens 55 Volumenprozent (Trockenbasis) beträgt.

15. Verfahren nach einem der Ansprüche 1 bis 14, dadurch gekennzeichnet, daß die Methanierungsreaktionen bei einem Druck von 3 bis 140 bar ausgeführt werden.

16. Verfahren nach einem der Ansprüche 1 bis 15, dadurch gekennzeichnet, daß der erste Strom vor der Ausführung der CO-Verschiebungs-Konversion oder vorläufigen Methanierung komprimiert wird.

17. Verfahren nach einem der Ansprüche 1 bis 16, dadurch gekennzeichnet, daß der Teil des zweiten Stromes, der das erste gesättigte Produkt bildet, vor dem gleichzeitigen Erhitzen und Sättigen komprimiert wird.

18. Verfahren nach einem der Ansprüche 1 bis 17, dadurch gekennzeichnet, daß mindestens drei Methanierungs-Reaktoren in jeder Stufe vorgesehen sind.

**Revendications**

1. Procédé pour produire des gaz riches en méthane par la méthanation, par conversion catalytique à la vapeur d'eau de CO, d'un gaz de synthèse contenant de l'hydrogène et au moins 30% en volume (sur base sèche) d'oxyde de carbone, lequel procédé comprend les étapes qui consistent:

(i) à diviser le gaz de synthèse en un premier courant mineur et un second courant majeur,

(ii) à mélanger ledit premier courant à de la vapeur d'eau pour former un corps réactionnel préliminaire dont la température est comprise dans la plage de 200 à 450°C et à soumettre ledit mélange réactionnel préliminaire à une méthanation par conversion catalytique de CO à la vapeur d'eau pour donner un produit réactionnel préliminaire,

(iii) à soumettre au moins une partie dudit second courant majeur simultanément à un chauffage et à une saturation en eau pour former un premier produit saturé,

(iv) à mélanger ledit produit réactionnel préliminaire à au moins une partie dudit premier produit saturé pour produire un courant réactionnel d'un premier stade dont la température est comprise dans la plage de 200 à 450°C,

(v) à soumettre ledit corps réactionnel du premier stade à une méthanation par conversion catalytique de CO à la vapeur d'eau de premier stade pour former un produit réactionnel de méthanation de premier stade,

(vi) à soumettre toute partie restante dudit second courant simultanément à un chauffage et à une saturation en eau pour former un second produit saturé,

(vii) à mélanger ledit produit réactionnel de premier stade à au moins une partie dudit second produit saturé pour former un courant réactionnel de second stade dont la température est comprise dans la plage de 200 à 450°C.

(viii) à soumettre ledit courant réactionnel de second stade à une méthanation catalytique de second stade pour former un produit réactionnel de méthanation de second stade.

2. Procédé selon la revendication 1, dans lequel ladite méthanation de premier stade comprend plusieurs stades de méthanation, en série, le produit du stade précédent étant mélangé à une partie dudit premier produit saturé pour former un mélange réactionnel pour le stade suivant, et dans lequel la température de chacun desdits mélanges réactionnel est comprise dans la plage de 200 à 450°C.

3. Procédé selon la revendication 1 dans lequel ladite méthanation de second stade comprend plusieurs stades de méthanation, en série, le produit du stade précédent étant mélangé à une partie dudit second produit saturé pour former un mélange réactionnel pour le stade suivant, et dans lequel la température de chacun desdits mélanges réactionnels est comprise dans la plage de 200 à 450°C.

4. Procédé selon la revendication 1, dans lequel ledit produit réactionnel de second stade est soumis à au moins une autre réaction de méthanation catalytique, la température dudit produit réactionnel de second stade, immédiatement avant ladite autre méthanation, n'étant pas supérieure à 500°C, pour former un produit réactionnel final.

5. Procédé selon la revendication 4, dans lequel de l'anhydride carbonique et, facultativement, de la vapeur d'eau sont retirés du produit du second stade avant ladite autre méthanation catalytique.

6. Procédé selon l'une quelconque des revendications précédentes, dans lequel ledit premier courant est soumis à une conversion de CO à la vapeur d'eau avant une méthanation préliminaire.

7. Procédé selon l'une quelconque des revendications précédentes, dans lequel ledit premier courant constitue 5 à 30% en volume dudit gaz de synthèse.

8. Procédé selon la revendication 7, dans lequel ledit premier courant constitue environ 20% en volume de gaz de synthèse.

9. Procédé selon l'une quelconque des revendications précédentes, dans lequel la température de l'eau utilisée pour simultanément chauffer et saturer lesdites parties du second courant est comprise entre 100 et 300°C.

10. Procédé selon la revendication 9, dans lequel au moins une partie de l'eau est chauffée par échange de chaleur direct ou indirect avec des courants à l'intérieur de l'enchaînement du procédé.

11. Procédé selon la revendication 10, dans lequel ladite eau est chauffée par échange de chaleur direct ou indirect avec un produit de réaction de méthanation.

12. Procédé selon l'une quelconque des revendications précédentes, dans lequel les produits de réaction de méthanation des premier et second stades sont refroidis avant d'être mélangés au produit saturé.

13. Procédé selon l'une quelconque des revendications précédentes, dans lequel le rapport vapeur d'eau/gaz sec des produits saturés est compris entre 0,1:1 et 1,0:1.

14. Procédé selon l'une quelconque des revendications précédentes, dans lequel la teneur en oxyde de carbone dudit gaz de synthèse est d'au moins 55% en volume (sur base sèche).

15. Procédé selon l'une quelconque des revendications précédentes, dans lequel lesdites réactions de méthanation sont conduites à une pression de 3 bars à 140 bars.

16. Procédé selon l'une quelconque des revendications précédentes, dans lequel ledit premier courant est comprimé avant l'exécution de la conversion de CO à la vapeur d'eau ou de la méthanation préliminaire.

17. Procédé selon l'une quelconque des revendications précédentes, dans lequel ladite partie dudit second courant formant ledit premier produit saturé est comprimée avant ledit chauffage et ladite saturation simultanés.

18. Procédé selon l'une des revendications précédentes, dans lequel au moins trois réacteurs de méthanation sont prévus dans chaque stade.

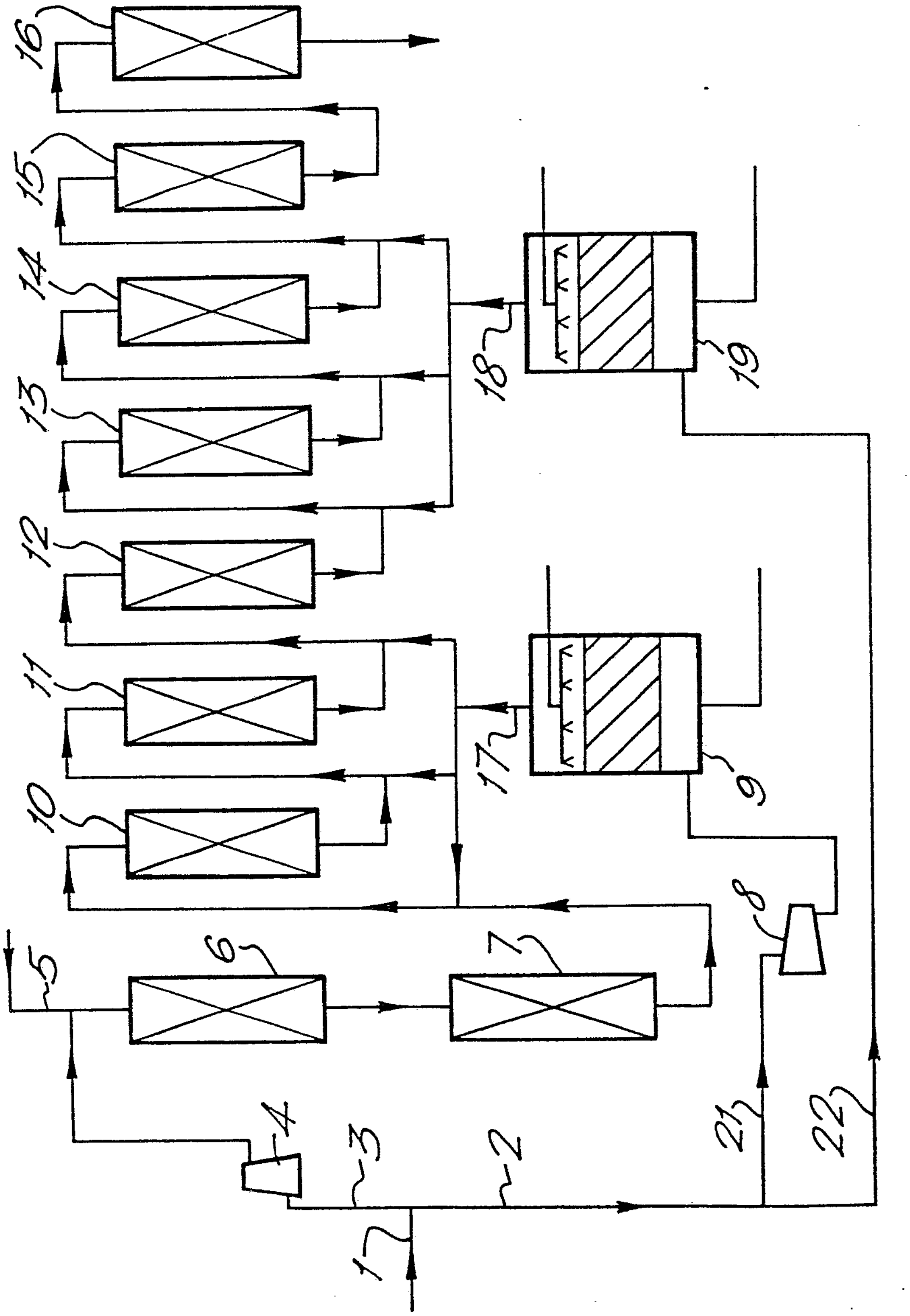
16
15
14
13
12
11
10
5
6
7
18
19
17
9
8
4
3
2
1
21
22